(19) Europäisches Patentamt
European Patent Office
Office européen des brevets

(11) Publication number: 0 230 946
A2

# (12) EUROPEAN PATENT APPLICATION

(21) Application number: 87100609.4

(51) Int. Cl.[4]: C 07 D 215/56

(22) Date of filing: 19.01.87

(30) Priority: 20.01.86 JP 9650/86
05.12.86 JP 291193/86

(43) Date of publication of application:
05.08.87 Bulletin 87/32

(84) Designated Contracting States:
BE CH DE FR GB IT LI NL SE

(71) Applicant: KYORIN PHARMACEUTICAL CO., LTD.
No. 5, Kanda Surugadai 2-chome
Chiyoda-ku Tokyo (JP)

(72) Inventor: Irikura, Tsutomu
no. 10-28, Ooizumigakuen-cho 7-chome
Nerima-ku Tokyo (JP)

Suzue, Seigo
no. 13-4, Aoba 4-chome
Kuki-shi Saitama-ken (JP)

Murayama, Satoshi
no. 6095, Tomonuma Nogi-machi
Shimotsuga-gun Tochigi-ken (JP)

Hirai, Keiji
no. 1-2-512, Aoba 1-chome
Kuki-shi Saitama-ken (JP)

Ishizaki, Takayoshi
9-6, Sakurada 4-chome Washimiya-machi
Kitakatsushika-gun Saitama-ken (JP)

(74) Representative: Patentanwälte TER MEER - MÜLLER - STEINMEISTER
Mauerkircherstrasse 45
D-8000 München 80 (DE)

## (54) Process for the preparation of quinolonecarboxylic acid derivatives.

(57) The present invention is concerned with certain novel process for the preparation of quinolonecarboxylic acid derivatives of the following formula.

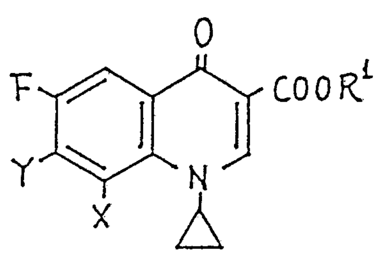

wherein $R^1$ is a lower alkyl group. X is a chlorine or bromine atom and Y is a halogen atom, which are useful intermediates for the synthesis of antibacterial agents.

EP 0 230 946 A2

**Description**

PROCESS FOR THE PREPARATION OF QUINOLONECARBOXYLIC ACID DERIVATES

Detailed description of the invention :

The present invention is concerned with certain novel process for the preparation of quinolonecarboxylic acid derivatives of the following formula (I),

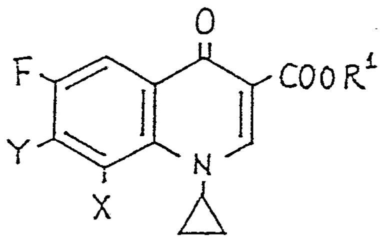

(I)

wherein $R^1$ is lower alkyl group, X is chlorine or bromine atom and Y is a halogen atom, which are useful intermediates for the synthesis of medicinal compounds, especially antibacterial agents.

We have found that the introduction of a chlorine or bromine atom at the 8-position in the antibacterial quinolonecarboxylic acid analogue gives rise to enhanced activity.

Thus, 8-bromo- or 8-chloro-1-cyclopropyl-6-fluoro-1,4-dihydro-7-substituted amino-4-oxo-3-quinolonecarboxylic acid derivatives were found to be useful antibacterial agent.

The detail of the process of the present invention is showed as follows;

The compound of the formula (II),

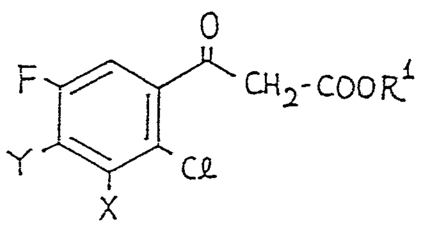

(II)

wherein $R^1$ is a lower alkyl group, X is a chlorine or bromine atom and Y is a halogen atom, is reacted with an equimolar or excess amount of orthoformic acid ester in acetic anhydride (1 to 20-fold volume per the total volume of the other reagents) at room temperature to 200 °C, preferably 100 to 150 °C for several hours to give the compound of the formula (III),

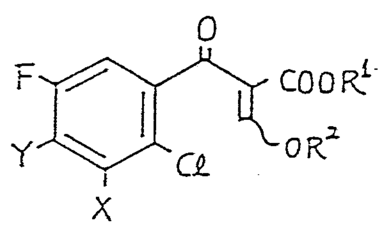

(III)

wherein $R^2$ is a lower alkyl group and $R^1$, X and Y have the above-stated meanings.

The compound of the formula (III) is converted by treating with cyclopropylamine (an equimolar or excess amount) in a sol vent such as ethanol to the compound of the formula (IV),

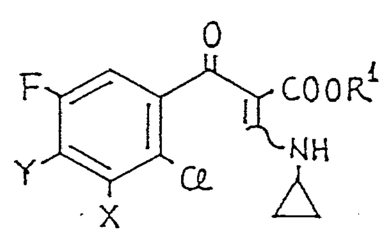

(IV)

wherein $R^1$. X and Y have the above-stated meanings.

The compound of the formula (IV) is treated with an appropriate base such as sodium fluoride, potassium fluoride or sodium hydride in a solvent such as dioxane, alcohol, dimethylformamide, dimethyl sulfoxide and sulfolane at 0 to 200 °C, preferably 50 to 150 °C for one or several hours to give the compound of the formula (I)

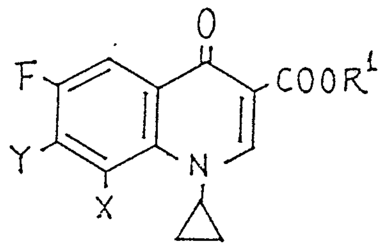

(I)

wherein $R^1$ and X have the above-stated meanings.

The starting compound of the fomrula (II) is also new and obtained, for example, from 3,4-difluoroaniline via several steps as illustrated in the following chart and in example.

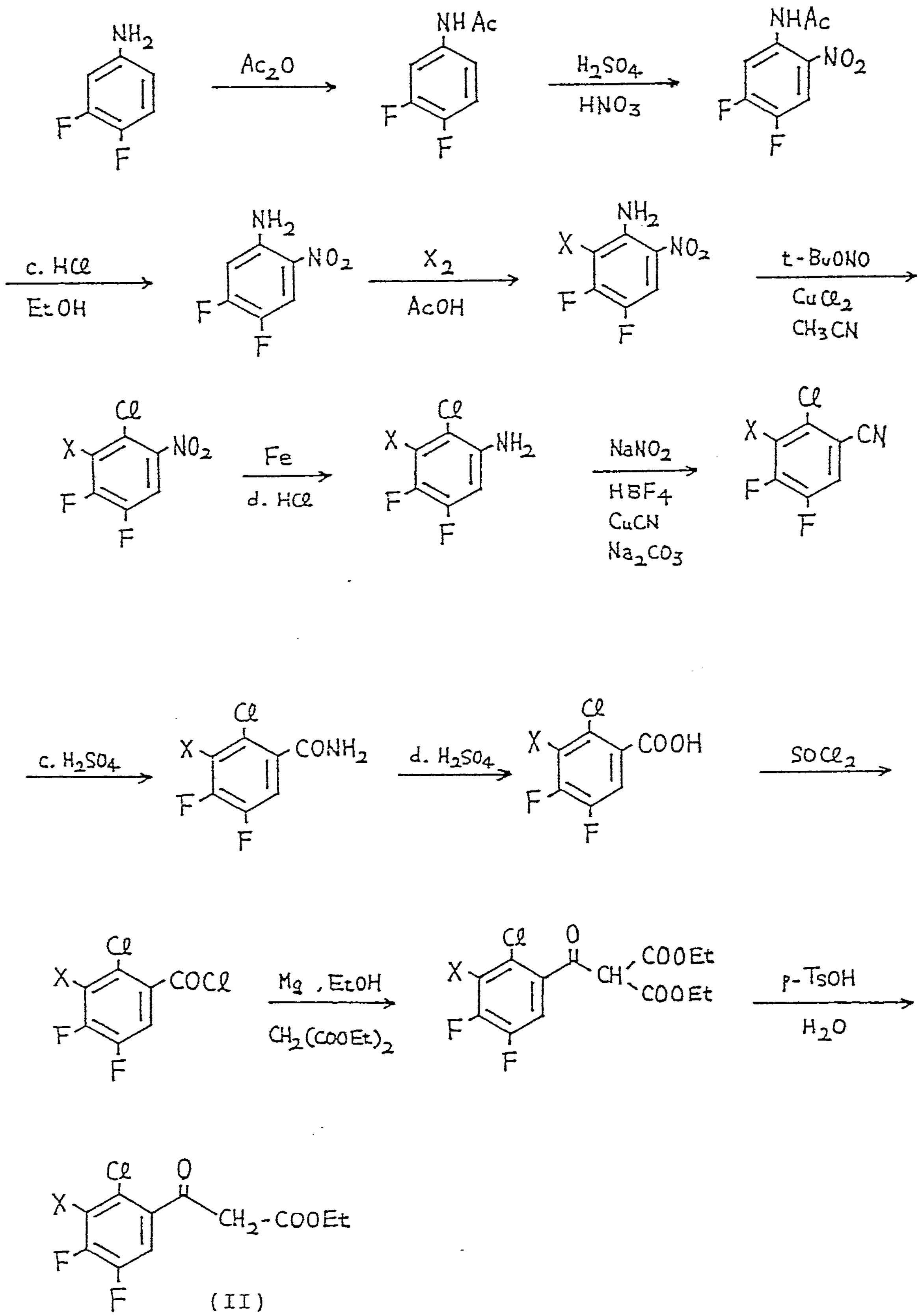


(II)

The following example will further illustrate the invention without, however, limiting it thereto.

Example 1 Ethyl 2-(2,3-dichloro-4,5-difluorobenzoyl)-3-ethoxyacrylate

A mixture of ethyl 2-(2,3-dichloro-4,5-difluorobenzoyl)-acetate (4.20 g), ethyl orthoformate (3.13 g) and acetic anhydride (3.60 g) was stirred at 130 to 140 °C for 3 hours and then concentrated to give the title compound (5.03 g) as yellow oil.

Example 2 Ethyl 2-(2,3-dichloro-4,5-difluorobenzoyl)-3-cyclopropylaminoacrylate

To a solution of ethyl 2-(2,3-dichloro-4,5-difluorobenzoyl)-3-ethoxyacrylate (5.03 g) in absolute ethanol (11 ml) was added a solution of cyclopropylamine (0.89 g) in absolute ethanol (7 ml) dropwise at 5 to 12 °C during 10 minutes. The mixture was allowed to stand below 5 °C for 30 minutes. The resulting precipitate was collected by filtration and washed with chilled ethanol to give the title compound (4.29 g) as white powdery crystals, mp 124-125 C.

Analysis (%) for $C_{15}H_{13}Cl_2F_2NO_3$, Calcd. (Found): C,49.48 (49.65); H, 3.60 (3.53); N, 3.85 (3.85).

Example 3 Ethyl 8-chloro-1-cyclopropyl-6,7-difluoro-1,4-dihydro-4-oxo-3-quinolinecarboxylate

To a solution of ethyl 2-(2,3-dichloro-4,5-difluorobenzoyl)-3-cyclopropylaminoacrylate (1.80 g) and anhydrous dioxane (5 ml) was added 55 % sodium hydride/oil (0.27 g) portionwise with stirring in an ice bath. The mixture was stirred for 30 minutes and then refluxed for an hour. After cooling, the reaction mixture was mixed with ice water (10 ml) and extracted with chloroform. The organic layer was washed with water, dried over anhydrous sodium sulfate and then concentrated. The residue was dissolved in ether, insoluble substance was filtered off and then the filtrate was concentrated. The resulting residue was recrystallized from methanol to give the title compound (0.83 g) as colorless flakes, mp 153-154 °C.

Analysis (%) for $C_{15}H_{12}ClF_2NO_3$, Calcd. (Found): C, 54.98 (55.03); H, 3.69 (3.54); N, 4.27 (4.28).

Example 4 Ethyl 2-(3-bromo-2-chloro-4,5-difluorobenzoyl)-3-ethoxyacrylate

A mixture of ethyl 3-bromo-2-chloro-4,5-difluorobenzoyl-acetate (4.55 g), ethyl orthoformate (2.96 g) and acetic anhydride (3.4 g) was stirred at 130 °C for 3 hours and then concentrated to give the title compound (5.23 g) as yellow oil.

Example 5 Ethyl 2-(3-bromo-2-chloro-4,5-difluorobenzoyl)-3-cyclopropylaminoacrylate

To a solution of ethyl 2-(3-bromo-2-chloro-4,5-difluorobenzoyl)-3-ethoxyacrylate (5.23 g) in absolute ethanol (15 ml) was added a solution of cyclopropylamine (0.85 g) in absolute ethanol (5 ml) at 3 to 5 °C during 18 minutes. After stirring for 2 hours, the mixture was concentrated. The residue was purified by silica gel chromatography eluting with dichloromethane-n-hexane (1:2) and further recrystallized from dichloromethane-n-hexane to give the title compound (3.65 g) as colorless flakes, mp 126-127 °C.

Analysis (%) for $C_{15}H_{13}BrClF_2NO_3$, Calcd. (Found): C, 44.09 (44.10); H, 3.21 (3.14); N, 3.43 (3.40).

Example 6 Ethyl 8-bromo-1-cyclopropyl-6,7-difluoro-1,4-dihydro-4-oxo-3-quinolinecarboxylate

To a suspension of 60 % sodium hydride/oil (0.5 g) in anhydrous dioxane (36 ml) was added ethyl 2-(3-bromo-2-chloro-4,5-difluorobenzoyl)-3-cyclopropylaminoacrylate (3.65 g) during 5 minutes. The mixture was stirred for an hour at room temperature and then poured into ice water (60 ml). The resulting precipitate was collected by filtration and washed with water sufficiently, then with ether and recrystallized from dichloromethane-n-hexane to give the title compound (2.42 g) as colorless prisms, mp 165.5-167 °C.

Analysis (%) for $C_{15}H_{12}BrF_2NO_3$, Calcd. (Found): C, 48.41 (48.61); H, 3.25 (3.32); N, 3.76 (3.76).

In example 1 and 4, the starting materials are synthesized by following process.

Reference example 1 N-(3,4-difluorophenyl)acetamide

To 3,4-difluoroaniline (28.2 g) was added acetic anhydride (30 ml) slowly. After allowed to stand for 30 minutes, the reaction mixture was poured into ice water (100 ml). The resulting precipitate was collected by filtration and washed with water sufficiently to give the title compound (34.7 g) as colorless needles, mp 127-127.5 °C.

Analysis (%) for $C_8H_7F_2NO$, Calcd. (Found): C, 56.14 (56.15); H, 4.12 (4.06); N, 8.18 (8.00).

Reference example 2 4,5-Difluoro-2-nitroaniline

To a solution of N-(3,4-difluorophenyl)acetamide (48 g) in concentrated sulfuric acid (140 ml) was added dropwise concentrated nitric acid (d 1.42, 56 ml) at -1 to 3 °C during 50 minutes with stirring in an ice-salt bath. After stirring for 1.5 hours at 3 to 16 °C, the reaction mixture was poured into ice water (560 ml). The resulting precipitate was collected by filtration, washed with chilled water sufficiently to give N-(4,5-difluoro-2-nitrophenyl)acetamide (55.3 g).

A solution of N-(4,5-difluoro-2-nitrophenyl)acetamide (70 g) in concentrated hydrochloric acid (110 ml) and ethanol (440 ml) was refluxed for 2 hours. The reaction mixture was poured into ice water (1.5 liter) and the resulting precipitate was collected by filtration and washed with chilled water sufficiently to give the title compound (53.2 g) as yellow prisms. mp 109-109.5 °C.

Analysis (%) for $C_6H_4F_2N_2O_2$, Calcd. (Found): C. 41.39 (41.29); H, 2.32 (2.31); N, 16.09 (15.96).

Reference example 3 2-Chloro-3,4-difluoro-6-nitroaniline

Into a solution of 4,5-difluoro-2-nitroaniline (20 g) in acetic acid (200 ml) was boubled chlorine gas at 13 to 15 °C during 45 minutes. The reaction mixture was poured into ice water (500 ml) and extracted with dichloromethane. The organic layer was washed with 6% aqueous sodium carbonate and with water successively, dried over anydrous sodium sulfate and then concentrated. The residue was purified with silica gel chromato graphy eluting with dichloromethane-n-hexane (1:4) and further recrystallized from hexane to give the title compound (2.91 g) as yellow needles. mp 86-88 °C.

Analysis (%) for $C_6H_3ClF_2N_2O_2$. Calcd. (Found): C, 34.56 (34.58); H. 1.45 (1.37); N, 13.43 (13.41).

Reference example 4 2,3-Dichloro-4,5-difluoronitrobenzene

To a mixture of anhydrous cupric chloride (2.2 g) and 2-chloro-3,4-difluoro-6-nitroaniline (2.63 g) in anydrous acetonitrile (20 ml) was added t-butylnitrite (2.0 g) dropwise at 46 to 55 °C during 9 minutes. After stirring for 13 minutes at the same temperature, the reaction mixture was poured into chilled 10 % diluted hydrochloric acid (20 ml) and extracted with benzene. The organic layer was washed with diluted hydrochloric acid and with water successively, dried over anhydrous sodium sulfate and concentrated. The resulting residue was purified by silica gel chromatography eluting with dichloromethane-n-hexane (1:5) to give the title compound (2.47 g), as yellow oil.

NMR (δ in $CDCl_3$), 7.75 (dd, J=7.0, 8.8 Hz)

Reference example 5 2,3-Dichloro-4,5-difluoroaniline

To a suspension of iron powder (1.9 g) in water (3 ml), with vigorous stirring at 50 to 60 °C, was added concentrated hydrochloric acid (0.4 ml) slowly. After mixed with ethanol (7 ml), 2,3-dichloro-4,5-difluoronitrobenzene (2.47 g) in ethanol (3 ml) was added dropwise to the suspension at 54 to 66 °C during 20 minutes. After stirring for 4 hours at the same temperature, the hot reaction mixture mixed with benzene was filtered and the insoluble materials were washed with hot ethanol (5 ml) and with benzene (20 ml) successively. The filtrate and washings were combined and mixed with ice water. The resulting organic layer was collected, washed with water, dried over anhydrous sodium sulfate and then concentrated. The resulting residue was purified by silica gel chromatography eluting with dichloromethane-n-hexane (1:3) to give the title compound (0.89 g) as colorless needles, mp 94-97.5 °C.

NMR (δ in $CDCl_3$), 6.52 (dd, J=7.0, 11.4 Hz)

Reference example 6 2,3-Dichloro-4,5-difluorobenzenediazonium tetrafluoroborate

To a suspension of 2,3-dichloro-4,5-difluoroaniline (0.88 g) in 42 % fluoroboric acid (10 ml) with stirring vigorously was added sodium nitrite (0.43 g) in water (1 ml) at -15 to 0 °C for 14 minutes. After stirred for 3 hours at the same temperature, the resulting precipitate was collected by filtration, washed with 42 % fluoroboric acid and then with ether to give the title compound (0.66 g) as white powdery crystals, mp 211 °C-(decompd.).

IR (KBr, $cm^{-1}$), 2300 (CN)

Reference example 7 2,3-Dichloro-4,5-difluorobenzonitrile

2,3-Dichloro-4,5-difluorobenzenediazonium tetrafluoroborate (0.65 g) was added portionwise during 10 minutes to a solution of cuprous cyanide (0.4 g), potassium cyanide (0.58 g) and sodium carbonate (0.12 g) in water (10 ml) with stirring vigorously at room temperature. After the mixture was stirred for 2.8 hours, benzene (20 ml) was added to the suspension and then the mixture was stirred for 15 minutes. The insoluble materials were collected by filtration, and washed with benzene. The filtrate and washings were combined and washed with water, dried over anhydrous sodium sulfate and then concentrated. The resulting residue was purified by silica gel chromatography eluting with dichloromethane-n-hexane (1:3) to give the title compound (0.15 g), mp 46.5-49.5 °C.

NMR (δ in $CDCl_3$), 7.49 (dd, J=7.3, 8.6 Hz)

IR (KBr, $cm^{-1}$), 2260 (CN)

Mass m/e; 207 ($M^+$), 209 ($M^+ + 2$), 211 ($M^+ + 4$), Calcd. MW. 207.99 for $C_7HCl_2F_2N$

Reference example 8 2,3-Dichloro-4,5-difluorobenzamide

A solution of 2,3-dichloro-4,5-difluorobenzonitrile (1.0 g) in concentrated sulfuric acid (2.5 ml) was stirred at 90 to 100 °C for 30 minutes and then cooled. The residue was poured into ice water and the resulting precipitate was collected by filtration and recrystallized from benzene to give the title compound (0.51 g) as colorless needles, mp 153-155 °C.

Analysis (%) for $C_7H_3Cl_2F_2NO$, Calcd. (Found): C, 37.20 (37.24); H, 1.34 (1.33); N, 6.20 (6.15).

Reference example 9 2,3-Dichloro-4,5-difluorobenzoic acid

A mixture of 2,3-dichloro-4,5-difluorobenzamide (0.5 g) and 18 N-sulfuric acid (2.5 ml) was stirred at 125 to 135 °C for 9 hours, and then poured into ice water. After standing overnight, the resulting precipitate was collected by filtration and recrystallized from hexane-benzene to give the title compound (0.3 g) as colorless prisms.

Analysis (%) for $C_7H_2Cl_2F_2O_2$, Calcd. (Found): C, 37.04 (37.23); H, 0.89 (0.93).

Reference example 10 2,3-Dichloro-4,5-difluorobenzoyl chloride

A solution of 2,3-dichloro-4,5-difluorobenzoic acid (9.3 g) and dimethylformamide (0.013 ml) in thionyl chloride (40 ml) was refluxed for 2.5 hours, and then concentrated. The resulting residue was purified by distillation in nitrogen atmosphere to give the title compound (8.7 g) as pale yellow oil, bp 123-126 °C/40 mmHg.

NMR (δ in $CDCl_3$), 7.89 (dd, J=7.5, 9.7 Hz)

Reference example 11 Diethyl 2-(2,3-dichloro-4,5-difluorobenzoyl)-malonate

Magnesium turnings (0.77 g) and carbon tetrachloride (0.4 ml) was added to absolute ethanol (5.3 ml). To the stirring suspension was added a solution of diethyl malonate (4.89 g) and absolute ethanol (8.5 ml) in toluene (21.3 ml) dropwise during 40 minutes at 20 to 40 °C. The mixture was stirred at 50 to 60 °C for 2 hours, and then cooled in an acetone-dry ice bath. A solution of 2,3-dichloro-4,5-difluorobenzoyl chloride (6.00 g) in anhydrous toluene (7.1 ml) was added dropwise to the resulting solution at -18 to -13 °C during 10 minutes. The mixture was warmed gradually to room temperature during 2 hours and then mixed with ice water (17 ml) containing concentrated sulfuric acid (1.1 ml). The resulting organic layer was collected and the water layer was extracted with toluene. The combined organic layer was washed with water, dried over anhydrous sodium sulfate and then concentrated to give the title compound (9.68 g) as pale yellow oil.

Reference example 12 Ethyl 2-(2,3-dichloro-4,5-difluorobenzoyl)acetate

To an emulsion of diethyl 2-(2,3-dichloro-4,5-difluorobenzoyl)malonate (9.71 g) in water (11.2 ml) was added p-toluene-sulfonic acid (11.2 mg) and refluxed for 3 hours with stirring vigorously. After cooling, the reaction mixture was extracted with dichloromethane. The organic layer was washed with 7 % aqueous sodium carbonate solution and then with water successively, dried over anhydrous sodium sulfate and concentrated. The residue was recrystallized from n-hexane to give the title compound (4.24 g) as powdery crystals, mp 52-53 °C.

Analysis (%) for $C_{11}H_8Cl_2F_2O_3$, Calcd. (Found): C, 44.47 (44.59); H, 2.71 (2.65).

Reference example 13 2-Bromo-3,4-difluoro-6-nitroaniline

Into a solution of 4,5-difluoro-2-nitroaniline (3.7 g) in acetic acid (27 ml) was added bromine (6.8 g) dropwise at 50 to 56 °C and stirred for 2.5 hours. The reaction mixture was poured into ice water (60 ml) and the resulting precipitate was col lected by filtration and washed with water sufficiently to give the title compound (4.7 g) as yellow prisms, mp 105 °C.

Analysis (%) for $C_6H_3BrF_2N_2O_2$, Calcd. (Found): C, 28.48 (28.62); H, 1.20 (1.15); N, 11.07 (11.00).

Reference example 14 3-Bromo-2-chloro-4,5-difluoronitrobenzene

To a mixture of anhydrous cupric chloride (3.1 g) and 2-bromo-3,4-difluoro-6-nitroaniline (4.6 g) in anhydrous acetonitrile (30 ml) was added t-butylnitrite (2.8 g) dropwise at 51 to 56 °C during 5 minutes. After stirring for 8 minutes at the same temperature, the reaction mixture was poured into chilled 10 % diluted hydrochloric acid (30 ml) and extracted with benzene. The organic layer was washed with diluted hydrochloric acid and with water successively, dried over anhydrous sodium sulfate and concentrated. The resulting residue was purified by silica gel chromatography eluting with dichloromethane-n-hexane (1:5) to give the title compound (4.0 g).

NMR (δ in $CDCl_3$), 7.79 (d, J=7.0, 8.8 Hz)

Reference example 15 3-Bromo-2-chloro-4,5-difluoroaniline

To a suspension of iron powder (38.6 g) in water (40 ml), with stirring vigorously at 50 to 60 °C, was slowly added concentrated hydrochloric acid (5 ml). After mixed with ethanol (100 ml), 3-bromo-2-chloro-4,5-difluoronitrobenzene (58.8 g) was added portionwise to the suspension at 60 to 75 °C during 30 minutes. After stirring for 70 minutes, the hot reaction mixture was filtered and the insoluble materials were washed with hot ethanol (30 ml) and with benzene (100 ml) successively. The filtrate and washings were combined and mixed with ice water. The resulting organic layer was washed with water, dried over anhydrous sodium sulfate and then concentrated. The resulting residue was purified by silica gel chromatography eluting with dichloromethane-n-hexane (1:1) to give the title compound (34.5 g) as light brown prisms, mp 83-86 °C.

Reference example 16 3-Bromo-2-chloro-4,5-difluorobenzenediazonium tetrafluoroborate

To a suspension of 3-bromo-2-chloro-4,5-difluoroaniline (30.1 g) in 42 % fluoroboric acid (180 ml) with stirring vigorously was added sodium nitrite (12 g) in water (30 ml) at -9 to 1 °C for 40 minutes. After stirred for 1.5 hours, the resulting precipitate was collected by filtration and washed with 42 % fluoroboric acid and then with ether to give the title compound (35.6 g) as light yellow needles, mp 300 °C.

IR (KBr, $cm^{-1}$): 2280 ($-N{\equiv}N^+$)

Reference example 17 3-Bromo-2-chloro-4,5-difluorobenzonitrile

3-Bromo-2-chloro-4,5-difluorobenzenediazonium tetrafluoroborate (35.6 g) was added portionwise during 40 minutes to a solution of cuprous cyanide (18.67 g), potassium cyanide (27.14 g) and sodium carbonate (5.52 g) in water (200 ml) with stirring vigorously at room temperature. After the mixture was stirred for 4.5 hours, benzene (250 ml) was added to the suspension and then the mixture was stirred for 25 minutes. The insoluble materials were collected by filtration, and washed with benzene. The filtrate and washings were combined and washed with water, dried over anhydrous sodium sulfate and then concentrated. The resulting residue was purified by silica gel chromatography eluting with dichloromethane-n-hexane (1:3) and further recrystallized from n-hexane-dichloromethane to give the title compound (10.9 g) as light yellow needles, mp 71-72.5 °C.

Analysis (%) for $C_7HBrClF_2N$. Calcd. (Found): C, 33.31 (33.22); H, 0.40 (0.31); N, 5.55 (5.48).

Reference example 18 3-Bromo-2-chloro-4,5-difluorobenzoic acid

A solution of 3-bromo-2-chloro-4,5-difluorobenzonitrile (9.8 g) in concentrated sulfuric acid (10 ml) was stirred at 100 °C for 35 minutes. After cooling, 18 N sulfuric acid (50 ml) and water (10 ml) were added to the reaction mixture. The reaction mixture was stirred at 100 °C for 4 hours and then poured into ice water (300 ml). The resulting precipitate was collected by filtration, washed with water sufficiently and recrystallized from dichloromethane-n-hexane to give the title compound (9.24 g) as colorless prisms, mp 137.5-139.5 °C.

Analysis (%) for $C_7H_2BrClF_2O_2$, Calcd. (Found): C, 30.97 (30.96); H, 0.74 (0.71).

Reference example 19 3-Bromo-2-chloro-4,5-difluorobenzoyl chloride

A solution of 3-bromo-2-chloro-4,5-difluorobenzoic acid (9.0 g) in thionyl chloride (33 ml) was refluxed for 2.5 hours, and then concentrated. The resulting residue was purified by distil lation to give the title compound (8.8 g), bp 109 °C/22 mmHg.

NMR (δ in $CDCl_3$), 7.93 (d, J=7.5, 9.7 Hz)

Reference example 20 Diethyl 2-(3-bromo-2-chloro-4,5-difluorobenzoyl)malonate

To the magnesium ethoxide (3.93 g) suspension was added a solution of diethyl malonate (4.65 g) in anhydrous toluene (30 ml) dropwise during 25 minutes at 20 to 25 °C. The mixture was stirred at 50 to 60 °C for 3 hours, and then cooled in an acetone-dry ice bath. A solution of 3-bromo-2-chloro-4,5-difluorobenzoyl chloride (7.65 g) in anhydrous toluene (15 ml) was added dropwise to the resulting solution at -16 to 0 °C during 25 minutes. The mixture was stirred for 2.5 hours below 0 °C, then further stirred at room temperature for 40 minutes and then mixed with ice water (15 ml) containing concentrated sulfuric acid (1 ml). The resulting organic layer was collected and the water layer was extracted with toluene. The combined organic layer was washed with water, dried over anhydrous sodium sulfate and then concentrated to give the title compound (11.44 g) as pale yellow oil.

Reference example 21 Ethyl 2-(3-bromo-2-chloro-4,5-difluorobenzoyl)-acetate

To an emulsion of diethyl 2-(3-bromo-2-chloro-4,5-difluorobenzoyl)malonate (11.44 g) in water (16 ml) was added p-toluene-sulfuric acid (15 mg) and refluxed for 3 hours with stirring vigorously. After cooling, the reaction mixture was extracted with dichloromethane. The organic layer was washed with water, dried over anhydrous sodium sulfate and concentrated. The residue was purified by silica gel chromatography eluting with dichloromethane-n-hexane (1:1) to give the title compound (4.56 g), mp 47-51 °C.

Analysis (%) for $C_{11}H_8BrClF_2O_3$, Calcd. (Found): C, 38.68 (37.94); H, 2.36 (2.19).

**Claims**

1. A process for the preparation of a compound of the formula (I),

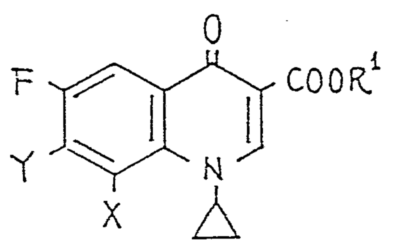

(I)

wherein $R^1$ is a lower alkyl group and X is a chlorine or bromine atom and Y is a halogen atom, which comprises condensing a compound of the formula (II),

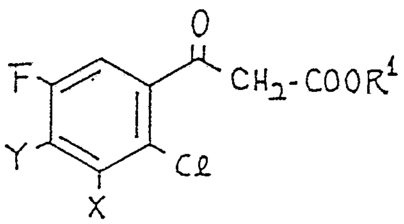

(II)

wherein $R^1$. X and Y have the above-stated meanings, with trialkyl orthoformate to give the compound of the formula (III),

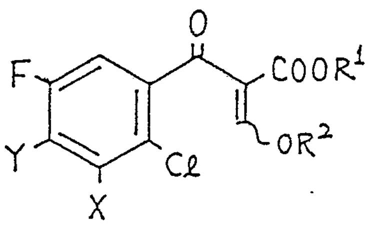

(III)

wherein $R^2$ is a lower alkyl group and $R^1$, X and Y have the above-stated meanings, reacting the compound of the formula (III) with cyclopropylamine to form the compound of the formula (IV),

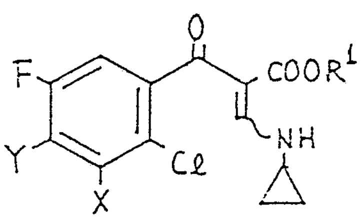

(IV)

wherein $R^1$, X and Y have the above-stated meanings, and cyclizing the compound of the formula (IV).